# EUROPEAN PATENT APPLICATION

(11) **EP 4 783 180 A1**
(43) Date of publication of application: **29.07.2026**
(21) Application number: 24882574.7
(22) Date of filing: 27.06.2024
(51) Int. Cl.: G16H 20/00, G16H 10/60, G16H 70/20, A61B 5/00, G16H 70/00

(54) **ELECTRONIC DEVICE FOR USER-CUSTOMIZED HEALTHCARE AND OPERATION METHOD THEREOF**

(30) Priority: 23.10.2023 KR 20230141857; 29.11.2023 KR 20230169023
(71) Applicant: Samsung Electronics Co., Ltd., Suwon-si, Gyeonggi-do 16677 (KR)
(72) Inventor: KIM, Jaepil, Suwon-si Gyeonggi-do 16677 (KR); YEO, Jaeyung, Suwon-si Gyeonggi-do 16677 (KR); PARK, Byeongju, Suwon-si Gyeonggi-do 16677 (KR); PARK, Jeongmin, Suwon-si Gyeonggi-do 16677 (KR); LEE, Myeongjin, Suwon-si Gyeonggi-do 16677 (KR); CHO, Sunghwan, Suwon-si Gyeonggi-do 16677 (KR)
(74) Representative: Gulde & Partner
(86) International application number: PCT/KR2024/008972
(87) International publication number: WO 2025/089542

(57) **Abstract**

Disclosed are an electronic device for user-customized healthcare and an operation method thereof. The electronic device may receive an access request from an external electronic device, and receive a user input for the access request. The electronic device may transmit personal information to the external electronic device based on the user input. The personal information may include personal device information for at least one device. The electronic device may receive device configuration information generated based on the personal information and a digital prescription from the external electronic device, and generate healthcare guide information related to the at least one device based on the device configuration information. The electronic device may display a user interface for guiding a health activity based on the healthcare guide information, and transmit a device control command for the at least one device to support the health activity. Besides, various embodiments understood through this document are also possible.

## Description

### [Technical Field]

The disclosure relates to an electronic device and an operation method thereof, for example, to the electronic device for user-customized healthcare and its operation method.

### [Background Art]

As use of a portable or mobile electronic device (e.g., a smartphone, a wearable electronic device, a mobile terminal, or a tablet personal computer (PC)) becomes common with recent advancement of mobile communication technology, services provided through the electronic device are gradually diversified. Such an electronic device, which is implemented in a form carried or worn by a user to closely relate to a user's daily life, may be effectively utilized for various services.

For example, the electronic device may provide a healthcare service for continuously monitoring biometric data or data related to workout, sleep and/or diet of the user and managing his/her health. The electronic device (e.g., a smartphone) may obtain user data from one or more sensors or an external electronic device (e.g., a wearable electronic device such as a smartwatch), and analyze a user health status based on the obtained user data. The electronic device may provide the healthcare service by use of the analysis results to assist the user in maintaining his/her health or to provide guidance (or coaching) for improving the user's health status.

### [Disclosure of Invention]

### [Solution to Problem]

An electronic device according to various embodiments may include communication circuitry, memory, a display, and at least one processor. The memory may store instructions, when executed by the at least one processor, causing the electronic device to receive an access request from an external electronic device via the communication circuitry, receive a user input for the access request, transmit personal information to the external electronic device via the communication circuitry based on the user input, the personal information including personal device information for at least one device, receive device configuration information generated based on the personal information and a digital prescription, from the external electronic device via the communication circuitry, generate healthcare guide information related to the at least one device based on the device configuration information, and display a user interface for guiding a health activity on the display based on the healthcare guide information, and transmit a device control command to the at least one device via the communication circuitry to support the health activity.

An external electronic device according to various embodiments may include communication circuitry, memory, and at least one processor. The memory may store instructions, when executed by the at least one processor, cause the external electronic device to, generate a digital prescription based on inputted medical information, transmit an access request to an electronic device via the communication circuitry, receive personal information from the electronic device via the communication circuitry, the personal information including personal device information for at least one device, generate device configuration information for a user-customized prescription based on the personal information and the digital prescription, and transmit the device configuration information to the electronic device via the communication circuitry.

An operation method of an electronic device according to various embodiments may include receiving an access request from an external electronic device, receiving a user input for the access request, transmitting personal information including personal device information for at least one device to the external electronic device based on the user input, receiving device configuration information generated based on the personal information and a digital prescription from the external electronic device, generating healthcare guide information related to the at least one device based on the device configuration information, and displaying a user interface for guiding a health activity based on the healthcare guide information, and transmitting a device control command to the at least one device to support the health activity.

An operation method of an external electronic device according to various embodiments may include generating a digital prescription based on inputted medical information, transmit an access request to the electronic device via the communication circuitry, receiving personal information from the electronic device via the communication circuitry, the personal information including personal device information for at least one device, generating device configuration information for a user-customized prescription based on the personal information and the digital prescription, and transmitting the device configuration information to the electronic device via the communication circuitry.

A computer-readable recording medium according to various embodiments may include may record a program for executing an operation method of an electronic device, which includes receiving an access request from an external electronic device, receiving a user input for the access request, transmitting personal information including personal device information for at least one device to the external electronic device based on the user input, receiving device configuration information generated based on the personal information and a digital prescription from the external electronic device, generating healthcare guide information related to the at least one device based on the device configuration information, and displaying a user interface for guiding a health activity based on the healthcare guide information, and transmitting a device control command to the at least one device to support the health activity.

A computer-readable recording medium according to various embodiments may include may record a program for executing an operation method of an external electronic device, which includes generating a digital prescription based on inputted medical information, transmit an access request to an electronic device via the communication circuitry, receiving personal information from the electronic device via the communication circuitry, the personal information including personal device information for at least one device, generating device configuration information for a user-customized prescription based on the personal information and the digital prescription, and transmitting the device configuration information to the electronic device via the communication circuitry.

### [Brief Description of Drawings]

FIG. 1 is a block diagram of an electronic device within a network environment according to an embodiment.
FIG. 2 is a diagram illustrating a simplified configuration of a healthcare system according to an embodiment.
FIG. 3A is a block diagram of an electronic device according to an embodiment.
FIG. 3B is a block diagram of a hospital server according to an embodiment.
FIG. 4 is a flowchart illustrating a user-customized healthcare method of a healthcare system according to an embodiment.
FIG. 5A is a flowchart illustrating an operation method of an electronic device according to an embodiment.
FIG. 5B is a flowchart illustrating an operation method of a hospital server according to an embodiment.
FIG. 6 is an example of a user interface for a user-customized healthcare function of an electronic device according to an embodiment.
FIG. 7 is another example of a user interface for a user-customized healthcare function of an electronic device according to an embodiment.

### [Mode for the Invention]

A healthcare service provided via an electronic device may be mostly provided by simply monitoring a user's activity (e.g., measurement, workout, or sleep) in daily life without expert intervention, evaluating monitoring results according to designated logic, and providing feedback to the user in the form of guidance based on the evaluation. In this case, expertise of guide details may be decreased.

Meanwhile, during a medical consultation at a hospital, since a doctor may not obtain user's baseline health information such as patient health status, daily activity level, related disease type beyond medical examination details or some personal information recorded in a hospital system, accuracy of prescription may decline or continuous monitoring and health management on the patient may not be achieved. In addition, even for patients presenting with similar symptoms, it may be necessary to apply different prescriptions depending on baseline health information of the individual patents.

It may be difficult for the patient to remember every detail of a prescribed activity (e.g., periodic medication, repetitive exercise for symptom improvement or sleeping habit adjustment) for a relatively long time (e.g., several days to several weeks) or to perform an appropriate activity at the right time. Such difficulty may be even greater for some patients (e.g., the elderly, young child, or a foreigner).

According to various embodiments of the disclosure, efficiency and convenience of the healthcare service may be enhanced by combining an electronic device owned by or easily accessible by the user (or the patient) in his/her daily live with prescription details.

According to various embodiments of the disclosure, personalized prescription details may be applied based on baseline health information of the individual user (or patient).

According to various embodiments of the disclosure, it is possible to improve expertise of guide content for the healthcare service through a verification procedure and to provide the user with the guide content based on prescription details in the form of an intuitive and easy user interface in daily life.

Effects obtainable from the disclosure are not limited to the above-mentioned effects, and other effects which are not mentioned may be clearly understood by those skilled in the art of the disclosure through the following descriptions.

Hereafter, the disclosure relates to an electronic device for user-customized healthcare and an operation method thereof.

Hereinafter, embodiments of the disclosure will be described in detail with reference to the drawings so that those skilled in the art may easily implement the disclosure. However, the disclosure may be implemented in various different forms and is not limited to the embodiments described herein. In connection with the description of the drawings, the same or similar reference numerals may be used for the same or similar components. In addition, in the drawings and related descriptions, descriptions on well-known functions and configurations may be omitted for clarity and brevity.

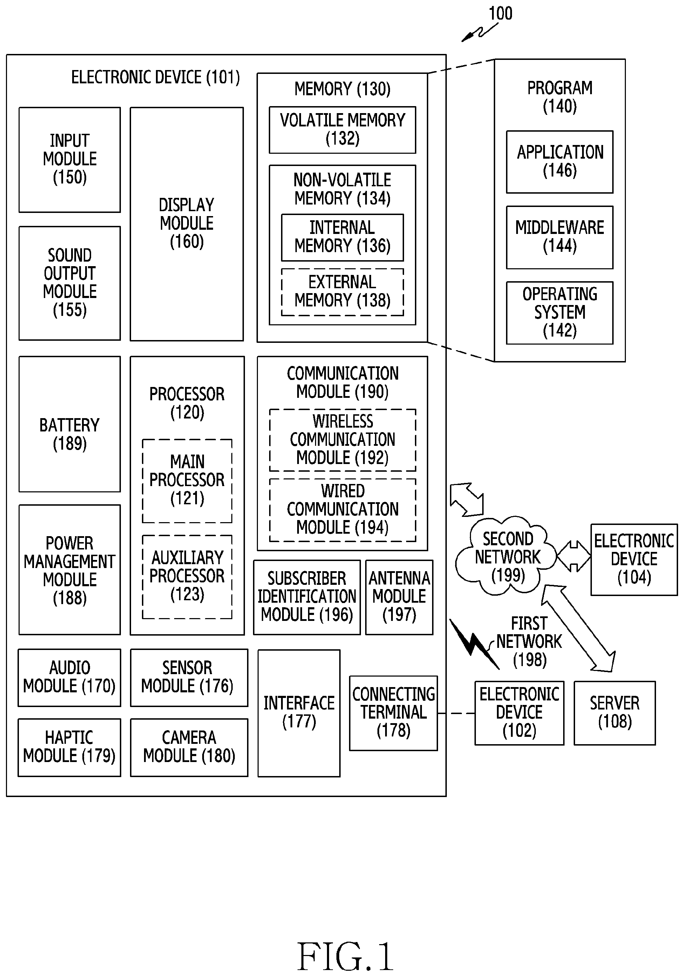

Fig. 1 is a block diagram illustrating an electronic device 101 in a network environment 100 according to various embodiments. Referring to Fig. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

Accoding to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

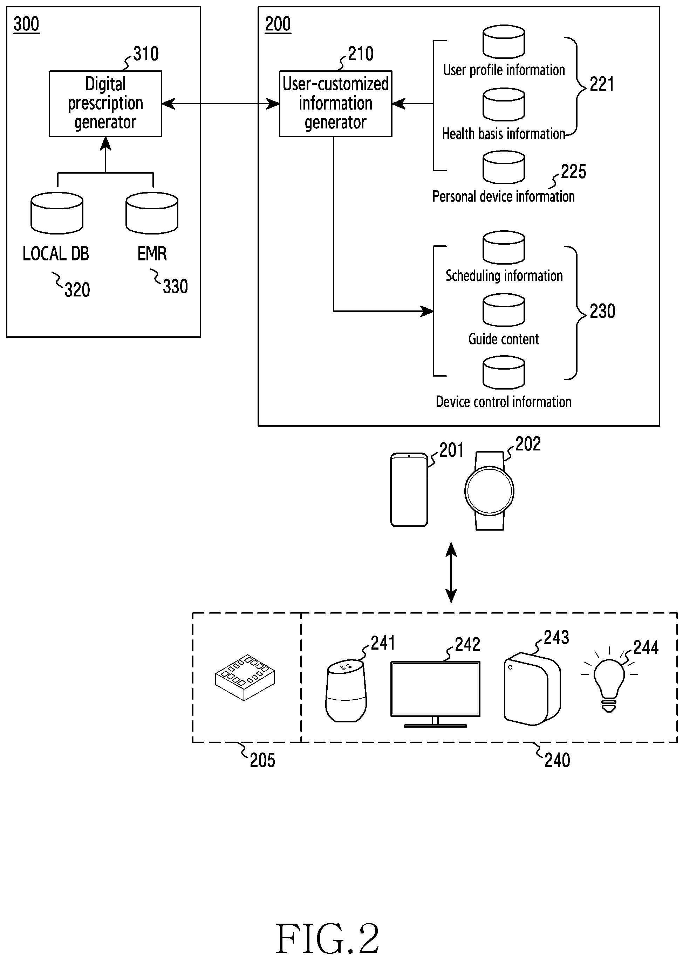

FIG. 2 is a diagram illustrating a simplified configuration of a healthcare system according to an embodiment.

Referring to FIG. 2, the healthcare system according to an embodiment may include an electronic device 200 (e.g., a wearable electronic device such as a smartphone 201 or a smartwatch 202) and an external electronic device 300 (e.g., a hospital server).

According to an embodiment, the electronic device 200 is an electronic device of a user (e.g., a patient visiting a hospital, or a patient remotely accessing the external electronic device 300), and may correspond to the electronic device 101 of FIG. 1.

According to an embodiment, the external electronic device 300 is an electronic device used by a doctor for medical examination, and may correspond to the server 108 or the electronic device 104 of FIG. 1.

For example, if the user of the electronic device 200 visits a hospital, the electronic device 200 and the external electronic device 300 may communicate with each other via the first network 198 (e.g., a short-range wireless communication network such as Bluetooth or Wi-Fi) of FIG. 1. For example, if the user of the electronic device 200 remotely accesses the external electronic device 300, the electronic device 200 and the external electronic device 300 may be communicated via the second network 199 (e.g., a long-range communication network such as cellular communication or the Internet) of FIG. 1.

According to an embodiment, the electronic device 200 may be a smartphone 201 carried by the user or a wearable electronic device (e.g., a smartwatch 202) worn by the user. The electronic device 200 may have a healthcare application installed to provide a user-customized healthcare function.

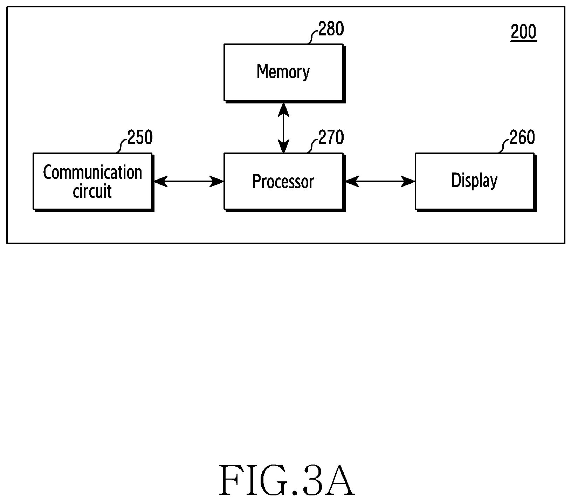

According to an embodiment, the electronic device 200 may include a user-customized information generator 210. According to an embodiment, the user-customized information generator 210 may include a hardware structure (e.g., a processor 270 of FIG. 3A) and/or a software structure (e.g., a healthcare application installed on the electronic device 200, a program or instructions stored in memory 280 of FIG. 3A).

According to an embodiment, the electronic device 200 may store personal information of the user. For example, the personal information may include personal health information 221 and personal device information 225.

According to an embodiment, the personal health information 221 may include user profile information and/or health basis information of the user. For example, the health basis information may include raw health data of the user collected in daily life. For example, the health basis information may include at least some of medical history data (e.g., presence of underlying conditions), health activity data (e.g., data such as walking, exercise, or calorie expenditure), health status data (e.g., a resting heart rate, an average heart rate, blood pressure, body temperature, or body composition), statistical information based on health activity data or health status data (e.g., activity or status evaluation based on same gender, same age group, or similar medical history), measurement trend data (e.g., measurement items, measurement cycle, measurement time per session, or measurement period).

According to an embodiment, the personal device information 225 may include information of at least one device registered in the healthcare application of the electronic device 200.

For example, the at least one device may include a device (e.g., the smartphone 201, the smartwatch 202, a sensor 205) carried or worn by the user, or a device (e.g., an IoT device 240) located at a place where the user resides or frequently visits. For example, the at least one device may include at least one of the electronic device 200 (e.g., one of the smartphone 201 and the smartwatch 202) and one or more other electronic devices (e.g., the other of the smartphone 201 and the smartwatch 202, the sensor 205, or the IoT device 240). For example, the at least one device may include a main device, and one or more peripheral devices for interworking with the main device.

For example, the at least one device may include one or more of the smartphone 201, the wearable electronic device (e.g., the smartwatch 202), the IoT device 240 (e.g., a speaker 241, a television 242, an air purifier 243, or a lamp 244), or the sensor 205. For example, the sensor 205 may be one or more sensors (e.g., a biometric sensor, a motion sensor, a temperature sensor, or a proximity sensor) included in the smartphone 201, the wearable electronic device (e.g., the smartwatch 202), or the IoT device 240. For example, the biometric sensor may include at least one of an electrode, a photoplethysmogram (PPG) sensor, a bioelectrical impedance analysis (BIA) sensor, an electrocardiography (ECG) sensor, and a galvanic skin response (GSR) sensor. The biometric sensor may measure user's biometric signals (e.g., signals such as bioimpedance, electrocardiogram, heart rate, blood pressure, blood oxygen saturation, stress, and/or galvanic skin response). For example, the motion sensor may include at least one of an accelerometer and a gyroscope.

For example, the main device for providing the user-customized healthcare function may be either the smartphone 201 or the wearable electronic device (e.g., the smartwatch 202). For example, the peripheral device for supporting the user-customized healthcare function may be at least one of the smartphone 201, the wearable electronic device (e.g., the smartwatch 202), the IoT device 240, or the sensor 205.

According to an embodiment, the external electronic device 300 may include a digital prescription generator 310. The external electronic device 300 may store a local database (DB) 320 and electronic medical records (EMR) 330.

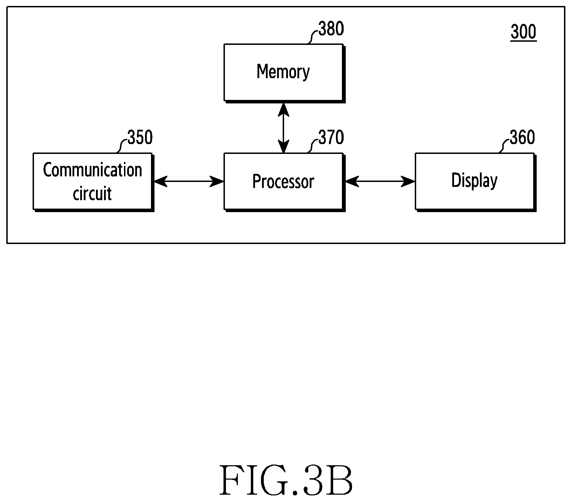

According to an embodiment, the digital prescription generator 310 may include a hardware structure (e.g., a processor 370 of FIG. 3B) and/or a software structure (e.g., programs, applications, or instructions stored in memory 380 of FIG. 3B).

In an embodiment, the digital prescription generator 310 and/or the user-customized information generator 210 each may be implemented in a form including a generative artificial intelligence (AI) model or in a form adopting a generative AI model. In some embodiments, the digital prescription generator 310 and the user-customized information generator 210 may be implemented to interwork with different external generative AI models respectively. Alternatively, the generative AI model of the digital prescription generator 310 and the generative AI model of the user-customized information generator 210 may be integrated.

According to an embodiment, if the user (or the patient) of the electronic device 200 visits a hospital or receives medical care from a doctor via remote access, the doctor may examine and diagnose the patient, and perform treatment if necessary. The external electronic device 300 may record medical information of corresponding treatment details in the EMR 330 according to doctor's inputs. The digital prescription generator 310 of the external electronic device 300 may generate (or issue) an initial digital prescription (or a primary digital prescription) based on the medical information recorded in the EMR 330 and basic data (e.g., clinical decision support system (CDSS)) in the local DB 320.

According to an embodiment, the user-customized information generator 210 of the electronic device 200 may be communicatively connected with the digital prescription generator 310 of the external electronic device 300, to transmit the personal information of the user to the digital prescription generator 310 through the communication connection. In an embodiment, the personal information may include the personal device information 225. In an embodiment, the personal information may further include the personal health information 221.

According to an embodiment, the digital prescription generator 310 of the external electronic device 300 may generate a secondary digital prescription based on the personal information received from the electronic device 200 and/or device configuration information for a user-customized prescription.

According to an embodiment, the digital prescription generator 310 of the external electronic device 300 may a digital prescription (or the secondary digital prescription) modified from the initial digital prescription (or the primary digital prescription) based on the personal information (e.g., the personal health information 221) received from the user-customized information generator 210 of the electronic device 200, by using a generative AI model. For example, the generative AI model may analyze patient's symptoms or prescription designated by the doctor, and the doctor's opinion through the initial digital prescription, and generate (or issue) the digital prescription (or the secondary digital prescription) for inducing the patient to perform various health activities (e.g., a measurement activity, an exercise activity, or a sleep activity) in daily life from the present to the next visit based on the analysis results and the personal health information 221.

According to an embodiment, the digital prescription generator 310 of the external electronic device 300 may utilize a generative artificial intelligence model, to generate device configuration information for providing a user-customized prescription based on the digital prescription (or the secondary digital prescription) and the personal information (e.g., the personal device information 225).

According to an embodiment, the generative AI model of the digital prescription generator 310 may identify one or more devices available for the health activity designated in the digital prescription among at least one device registered in the healthcare application of the electronic device 200, based on the personal device information 225 received from the user-customized information generator 210. The generative AI model may generate the device configuration information (e.g., prompt information specifying device-specific execution commands) for the identified one or more devices. For example, the generated device configuration information may include target item setup information related to at least one device (e.g., at least one of the smartphone 201, the wearable electronic device (e.g., the smartwatch 202), or the sensor 205) registered in the healthcare application of the electronic device 200. For example, the target item setup information may include setup information for a measurement item (e.g., a step count, a heart rate, a blood pressure, body temperature, or body composition) to be managed or monitored through the healthcare application installed on the electronic device 200 from the present until the next medical treatment, a reference value (e.g., a target value) related to each measurement item, a measurement cycle, a measurement frequency, a measurement time for one measurement, a measurement period, or a concurrent measurement item. The generated device configuration information may be transmitted to the user-customized information generator 210 of the electronic device 200.

According to an embodiment, the external electronic device 300 may display the device configuration information generated by the digital prescription generator 310 for the doctor to view, and transmit the device configuration information to the electronic device 200 in response to an approval input of the doctor with respect to the device configuration information.

According to an embodiment, the user-customized information generator 210 of the electronic device 200 may generate healthcare guide information 230 related to at least one device registered in the healthcare application of the electronic device 200, based on the device configuration information received from the digital prescription generator 310 of the external electronic device 300. The user-customized information generator 210 may provide the healthcare guide information 230 to the user to guide the user's health activity according to the digital prescription. The user-customized information generator 210 may control at least one device registered in the healthcare application of the electronic device 200 based on the healthcare guide information 230 to support the user's health activity according to the digital prescription.

According to an embodiment, the user-customized information generator 210 may generate the healthcare guide information 230 using a generative AI model. The healthcare guide information 230 generated by the user-customized information generator 210 may include at least part of scheduling information related to the user's health activity (e.g., scheduling information for measurement, exercise, or sleep activity), guide content (e.g., images, videos, or audio representing advice, guidance, or coaching messages for measurement, exercise, or sleep activity), or device control information. The device control information (e.g., prompt information specifying device-specific execution commands) may correspond to the device configuration information received from the digital prescription generator 310. For example, the device control information may be identical to the device configuration information, or may be processed or updated from the device configuration information.

In some embodiments, the device configuration information generated by the digital prescription generator 310 may be readjusted by the user-customized information generator 210 and fed back to the digital prescription generator 310 of the external electronic device 300. For example, if the patient who is the user of the electronic device 200 does not consent to sharing his/her personal information with the external electronic device 300, the user-customized information generator 210 may readjust (e.g., filter) the device configuration information based on the personal information stored on the electronic device 200, and then feed the readjusted device configuration information back to the digital prescription generator 310 of the external electronic device 300. The device configuration information fed back may be finally approved by the digital prescription generator 310 of the external electronic device 300 or by the doctor.

FIG. 3A is a block diagram of an electronic device 200 according to an embodiment.

Referring to FIG. 3A, the electronic device 200 is an electronic device for a user (e.g., a patient visiting a hospital, or a patient remotely accessing an external electronic device 300), and may include communication circuitry 250, a display 260, a processor 270, and memory 280. The electronic device 200 of FIG. 3A may correspond to the electronic device 101 shown in FIG. 1 or the electronic device 200 shown in FIG. 2. For example, the electronic device 200 may be a smartphone 201 carried by the user or a wearable electronic device (e.g., a smartwatch 202) worn by the user.

According to an embodiment, the electronic device 200 may have a healthcare application installed therein to provide a user-customized healthcare function.

In an embodiment, the communication circuitry 250 (e.g., the communication module 190 of FIG. 1) may establish a communication connection with one or more external electronic devices (e.g., the electronic device 104 of FIG. 1, the server 108 of FIG. 1, or the external electronic device 300 of FIG. 2), and transmit and receive various data. For example, the communication circuitry 250 may support at least one communication scheme of cellular communication, the Internet, Wi-Fi, Bluetooth, near field communication (NFC), or ultra-wide band (UWB) communication.

In an embodiment, the processor 270 (e.g., the processor 120 of FIG. 1) may execute an application and control various hardware by executing code or instructions written in a programming language stored in the memory 280 of the electronic device 200. As the code stored in the memory 280 is executed, the processor 270 may be driven to perform a designated function (or logic). Alternatively, the processor 270 may execute instructions stored in the memory 280 to perform a designated function (or logic).

In an embodiment, the memory 280 (e.g., the memory 130 of FIG. 1) may store various data used by at least one component (e.g., the processor 270 or the communication circuitry 250) of the electronic device 200. The data may include, for example, software (e.g., a program, an application, or instructions), and input or output data related to commands associated therewith.

In an embodiment, the memory 280 (e.g., the memory 130 of FIG. 1) may store instructions for, when executed, controlling the processor 270 to perform various operations.

In an embodiment, the display 260 (e.g., the display module 160 of FIG. 1) may visually provide (or display) information to outside (e.g., the user) of the electronic device 200. In an embodiment, the display 260 may include a touch sensor configured to detect touch, and/or a pressure sensor configured to measure the magnitude of force generated by the touch.

According to an embodiment, the processor 270 (e.g., the processor 120 of FIG. 1) may include at least one processor. The processor 270 may receive an access request from the external electronic device 300 via the communication circuitry 250. The processor 270 may receive a user input for the access request and, based on the user input, transmit the user's personal information to the external electronic device 300 via the communication circuitry 250. The personal information may include personal device information (e.g., the personal device information 225 of FIG. 2) for at least one device of the user. The personal information may further include personal health information (e.g., the personal health information 221 of FIG. 2) of the user.

According to an embodiment, the processor 270 may receive device configuration information generated based on the personal information and digital prescription of the user, from the external electronic device 300 via the communication circuitry 250. The processor 270 may generate healthcare guide information (e.g., the healthcare guide information 230 of FIG. 2) related to at least one device based on the device configuration information.

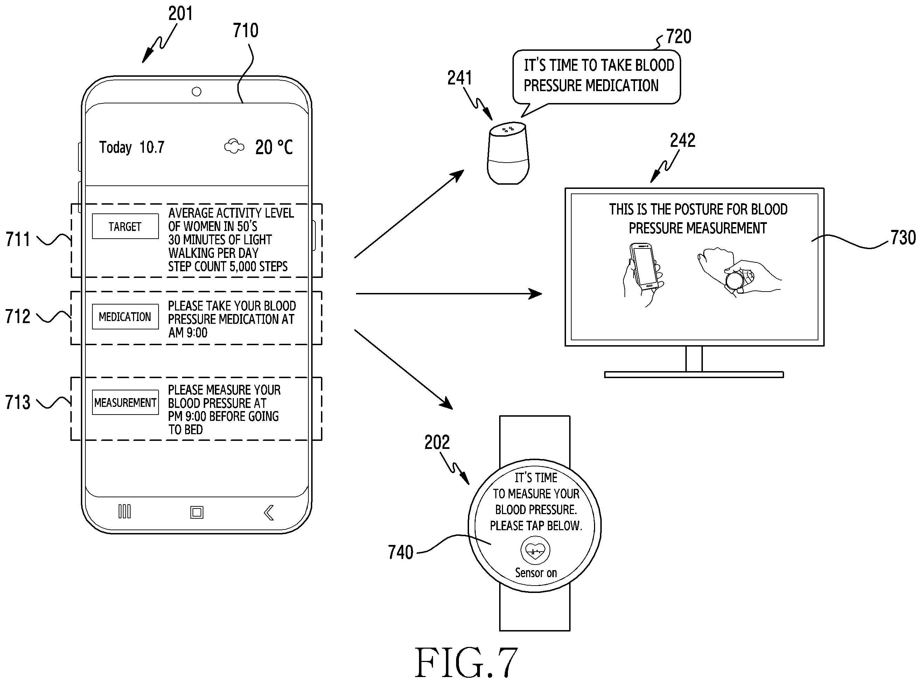

According to an embodiment, based on the healthcare guide information, the processor 270 may display a user interface (e.g., a first screen 710 of FIG. 7) for guide the user's health activity through the display 260. The processor 270 may transmit a device control command to at least one device (e.g., a smartwatch 202, a speaker 241, or a television 242 of FIG. 7) via the communication circuitry 250 to support the user's health activity.

According to an embodiment, at least one device of the user used for the user-customized healthcare function may include at least one of the electronic device 200 (e.g., one of the smartphone 201 and the smartwatch 202) and one or more other electronic devices (e.g., the other of the smartphone 201 and the smartwatch 202, the sensor 205, or the IoT device 240).

According to an embodiment, at least one device of the user used for the user-customized healthcare function may include a main device and one or more peripheral devices.

According to an embodiment, at least one device used for the user-customized healthcare function may include one or more of the smartphone 201, the wearable electronic device (e.g., the smartwatch 202), an IoT device (e.g., the speaker 241, the television 242, the air purifier 243, the lamp 244), or the sensor 205.

According to an embodiment, the user interface showing the healthcare guide information may include information related to one or more of a measurement activity, an exercise activity, a sleep activity, scheduling, or guide content associated with the health activity. The device control command transmitted to at least one device of the user may be to instruct a function to be executed on each of the at least one device to support the user's health activity.

According to an embodiment, based on scheduling information included in the healthcare guide information, the processor 270 may repeatedly (e.g., periodically, or every time a designated event occurs) display the user interface showing the healthcare guide information and transmit the device control command to at least one device of the user, for a designated period (e.g., from the present until the next medical treatment).

According to an embodiment, the processor 270 may display a user interface guiding initial health activities of the user. The processor 270 may collect actual health activity information of the user using at least one of at least one device of the user. Based on the collected actual health activity information, the processor 270 may update the healthcare guide information and/or the user interface showing the healthcare guide information. The processor 270 may display the updated user interface and transmit a device control command related to the updated user interface to at least one device of the user.

According to an embodiment, the processor 270 may display a user interface inquiring about a privacy exposure level (e.g., whether to protect privacy, or a privacy protection level) on the display 260. For example, the user interface may include a text and/or an image.

In an embodiment, the user interface inquiring about the privacy exposure level may be implemented in at least one type of a visual type (e.g., a screen), an auditory type (e.g., audio, sound), and a tactile type (e.g., vibration). For example, the processor 270 may output the user interface in a manner such as a pop-up window, a window (execution) screen, a sound notification, or a vibration notification using the display 260, the audio module 170, and the haptic module 179.

According to an embodiment, based on a user input to the user interface inquiring about the privacy exposure level, the processor 270 may transmit only some information filtered based on the privacy exposure level from the personal information stored in the electronic device 200, to the external electronic device 300.

According to an embodiment, the processor 270 may identify whether the electronic device 200 is located at a designated location (e.g., home). If the electronic device 200 is located at the designated location, the processor 270 may display a user interface presenting the healthcare guide information and transmit a device control command for at least one device of the user.

According to an embodiment, the processor 270 may identify whether it is necessary to use an additional device not included in at least one device of the user based on the device configuration information received from the external electronic device 300. If the use of the additional device is required, the processor 270 may search for a place available with the additional device. The processor 270 may display an indicator guiding to the searched place within the user interface representing the healthcare guide information.

FIG. 3B is a block diagram of the external electronic device 300 (e.g., a hospital server) according to an embodiment.

Referring to FIG. 3B, the external electronic device 300 is an electronic device used by the doctor for a medical consultation, and may include communication circuitry 350, a processor 370, and memory 380. The external electronic device 300 may further include a display 360.

The external electronic device 300 of FIG. 3B may correspond to the server 108 of FIG. 1, the electronic device 104 of FIG. 1, or the external electronic device 300 of FIG. 2.

In an embodiment, the display 360 may visually provide (or display) information to outside (e.g., a doctor) of the external electronic device 300.

In an embodiment, the processor 370 may executes an application and control various hardware by executing code or instructions written in a programming language stored in the memory 380 of the external electronic device 300. As code stored in the memory 380 is executed, the processor 370 may be driven to perform a designated function (or logic). Alternatively, the processor 370 may execute instructions stored in the memory 380 to perform a designated function (or logic).

According to an embodiment, the processor 370 may include at least one processor.

In an embodiment, the memory 380 may store instructions for, when executed, controlling the processor 370 to perform various operations.

According to an embodiment, the processor 370 may generate an initial digital prescription based on medical information entered by the doctor. The processor 370 may transmit an access request to the electronic device 200 of the user via the communication circuitry 350. The processor 370 may receive the personal information of the user from the electronic device 200 via the communication circuitry 350. The personal information may include personal device information (e.g., the personal device information 225 of FIG. 2) for at least one device. The personal information may further include personal health information (e.g., the personal health information 221 of FIG. 2) of the user.

According to an embodiment, the processor 370 may generate a digital prescription (e.g., a secondary digital prescription) modified from the initial digital prescription (e.g., the primary digital prescription) based on the user's personal information received from the electronic device 200. The processor 370 may generate device configuration information for a user-customized prescription based on the digital prescription. The processor 370 may transmit the generated device configuration information to the electronic device 200 via the communication circuitry 350.

According to an embodiment, the processor 370 may determine whether any essential device required for the prescription is missing based on the digital prescription and the personal device information of the user. If the essential device is missing, the processor 370 may add information of the essential device to the device configuration information to be transmitted to the electronic device 200.

For example, the processor 370 may identify based on the digital prescription that the user of the electronic device 200 is a diabetic patient requiring precise blood glucose measurement using a dedicated blood glucose meter at least twice a week. Based on the personal device information of the user, the processor 370 may identify that the smartphone 201 or the smartwatch 202 of the user has no sensor meeting functional specifications required for the precise blood glucose measurement. In this case, the processor 370 may add to the device configuration information, information of the dedicated blood glucose meter and/or information notifying of the precise blood glucose measurement required at least twice a week.

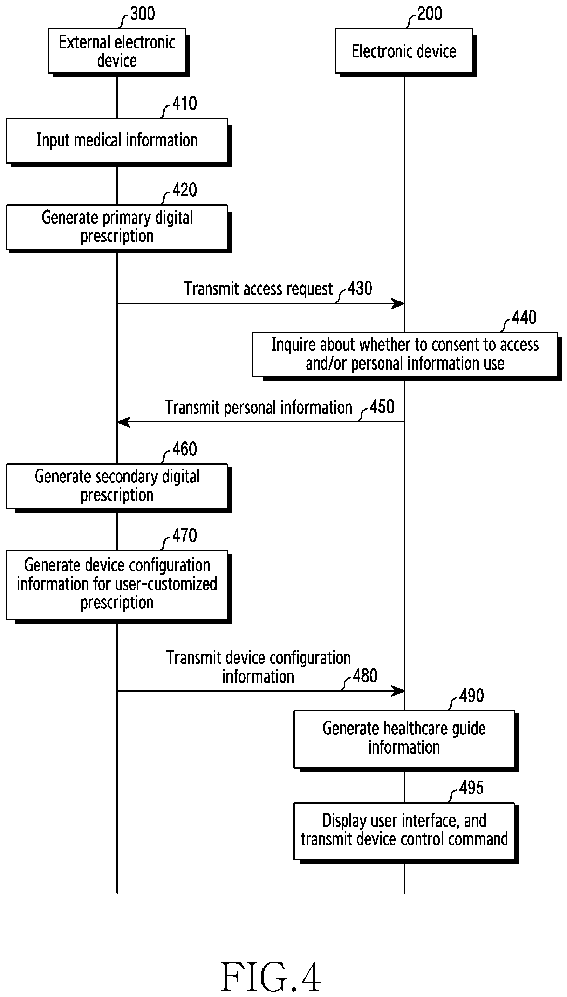

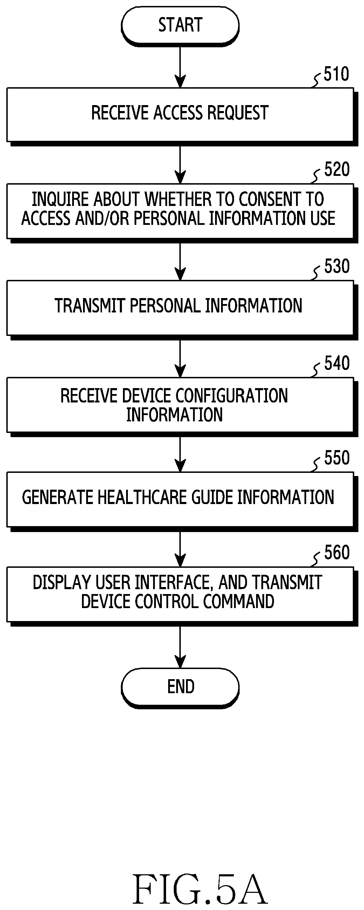

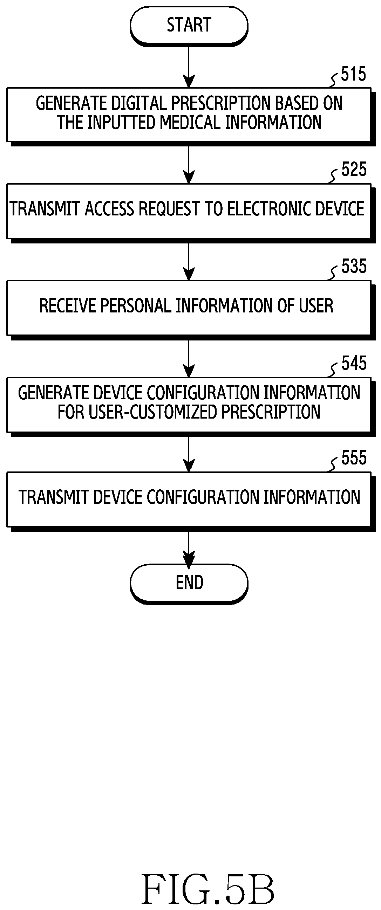

Hereafter, operation methods of an electronic device for user-customized healthcare according to various embodiments are described with reference to FIG. 4, FIG. 5A, and FIG. 5B. Operations shown in the embodiments of FIG. 4, FIG. 5A, and FIG. 5B may be performed sequentially, but are not necessarily performed in sequence. For example, the order of the operations may be changed, and at least two operations may be performed in parallel. Alternatively, at least one of the illustrated operations may be omitted, the order of some operations may be changed, some operations may be combined, or other operation may be added. At least some of the operations of the operation methods of the electronic device for the user-customized healthcare according to the various embodiments to be described may be performed in correspondence with or in combination with each other.

FIG. 4 is a flowchart illustrating a user-customized healthcare method of a healthcare system according to an embodiment.

The healthcare system according to an embodiment may include an electronic device 200 and an external electronic device 300.

Referring to FIG. 4, the user-customized healthcare method of the healthcare system according to an embodiment may include operation 410, operation 420, operation 430, operation 440, operation 450, operation 460, operation 470, operation 480, operation 490, and operation 495.

In operation 410, the external electronic device 300 may record into the EMR 330, medical information related to medical records (e.g., details on medical examinations, diagnosis results, and treatments) generated during the doctor's consultation with the patient who is the user of the electronic device 200 (e.g., the smartphone 201), based on inputs from the doctor.

For example, if the user of the electronic device 200 (e.g., the smartphone 201) is a 48-year-old male patient and visits the hospital due to a headache lasting for two weeks upon waking in the morning, the doctor may conduct medical examinations regarding the patient's symptoms and lifestyle, and discover from last-year health checkup recorded on the EMR 330 of the external electronic device 300 that the patient has borderline hypertension. If the doctor measures the blood pressure and determines hypertension with the blood pressure result of 145/92 mmHg, the doctor may diagnose the headache as a symptom caused by the hypertension and input the corresponding diagnosis result into the EMR 330.

In operation 420, the external electronic device 300 may generate an initial digital prescription (or a primary digital prescription), based on the medical information recorded in the EMR 330 in operation 410 and the local DB 320 (e.g., a CDSS).

For example, if the doctor inputs a prescription generation request on the screen of the external electronic device 300, the digital prescription generator 310 of the external electronic device 300 may automatically set and display a default template on the screen. The default template may reflect the medical information (e.g., medical examinations, diagnostic results, treatment details) inputted by the doctor.

For example, the primary digital prescription may be a file containing prescription details, and may include medication information (e.g., blood pressure medication to be taken twice a day for two weeks), and/or target item setup information required for monitoring and managing the patient's health status (e.g., blood pressure management required, a current blood pressure value, or a target blood pressure range).

In operation 430, the external electronic device 300 may transmit an access request to the electronic device 200 (e.g., the smartphone 201) of the patient.

For example, the doctor may identify whether the patient possess his/her wearable electronic device, and if the patient possesses a wearable electronic device, may request a connection between the external electronic device 300 and the electronic device 200 (e.g., the smartphone 201) through the external electronic device 300.

In operation 440, the electronic device 200 (e.g., the smartphone 201) may display a user interface inquiring about whether the user consents to the access and/or personal information use by the external electronic device 300 on the screen of the electronic device 200 in response to the access request from the external electronic device 300. If the patient consents to the access and/or the use of the personal information by the external electronic device 300 in response to the user interface displayed on the screen of the electronic device 200, operation 450 may be proceeded. In operation 450, the electronic device 200 may process the personal information stored locally on the electronic device 200 into a designated file format and transmit it to the external electronic device 300. The personal information may include personal device information (e.g., a model name of the wearable electronic device) of at least one device of the patient. The personal information may further include personal health information (e.g., user profile information, and/or health basis information).

In operation 460, the external electronic device 300 may generate a digital prescription (or a secondary digital prescription) modified from the initial digital prescription (or the primary digital prescription) generated in operation 420 based on the personal information (e.g., personal health information) received from the electronic device 200 (e.g., the smartphone 201).

For example, the digital prescription generator 310 of the external electronic device 300 may generate the secondary digital prescription, based on prestored fitness and medical knowledge data and the personal health information of the patient received from the electronic device 200.

For example, the secondary digital prescription is a file which records modifications, adjustments, or updates made to the primary digital prescription, and may include adjusted medication information (e.g., adjustment of blood pressure medication ingredients based on a patient's recent health status or medical history), and/or adjusted target item setup information (e.g., setup information on blood pressure management required, a current blood pressure value, a target blood pressure range, and a recommended health activity considering age, gender, medical history, recent health status, or average activity level of the patient).

In operation 470, the external electronic device 300 may generate device configuration information for a user-customized prescription based on the secondary digital prescription generated in operation 460 and the personal information (e.g., the personal device information) received in operation 450.

For example, the digital prescription generator 310 of the external electronic device 300 may generate the device configuration information based on the secondary digital prescription and the personal device information. The device configuration information may include the target item setup information for monitoring and/or managing the patient's health activities in daily life.

For example, the digital prescription generator 310 of the external electronic device 300 may identify functional specifications of the smartwatch 202 which is the wearable electronic device possessed by the patient, or a type of sensor(s) embedded in the smartwatch 202, based on the personal device information (e.g., the model name of the smartwatch 202) received from the electronic device 200 (e.g., the smartphone 201). The digital prescription generator 310 may generate the device configuration information based on the identified functional specifications or the type of the sensor(s).

For example, the device configuration information may include target item setup information related to a measurement item, a reference value (e.g., a target value) related to each measurement item, a measurement cycle, a measurement frequency, a measurement duration per session, a measurement period, or a concurrent measurement item. For example, the measurement item may set up a step count, a heart rate, heart rate variability (HRV), blood pressure, body temperature, body composition, or sleep quality. For the heart rate or the HRV, the measurement cycle (e.g., once every 10 minutes) and the measurement duration (e.g., 1 minute per measurement) may be set up. For the step count, a daily target step count (e.g., 5,000 steps) may be set up. For the body composition, the measurement cycle (e.g., once every two weeks) may be set up. For the sleep quality, a sleep start time or a sleep duration may be set up.

In operation 480, the external electronic device 300 may transmit the device configuration information generated in operation 470 to the electronic device 200 (e.g., the smartphone 201) of the patient.

In operation 490, the electronic device 200 (e.g., the smartphone 201) may generate healthcare guide information related to at least one device (e.g., the smartwatch 202) of the patient based on the device configuration information received from the external electronic device 300.

In operation 495, the electronic device 200 may display a user interface (e.g., the first screen 710 of FIG. 7) for guiding the patient's health activities based on the healthcare guide information generated in operation 490, and transmit a device control command for at least one device (e.g., a smartwatch 202, a speaker 241, or a television 242 of FIG. 7) of the patient to support the health activities.

If the patient does not consent to the connection of the external electronic device 300 and/or the use of the personal information in operation 440, the patient personal information stored on the electronic device 200 may not be transmitted to the external electronic device 300. Accordingly, in operation 480, the patient personal information may not be reflected in generating the device configuration information to be provided to the electronic device 200. In this case, the user-customized information generator 210 of the electronic device 200 may readjust (e.g., filter) the device configuration information received from the external electronic device 300 based on the patient personal information stored on the electronic device 200.

For example, the primary device configuration information (e.g., the device configuration information related to the step count, the body composition, or the sleep quality) generated by the digital prescription generator 310 using the initial digital prescription and the personal information stored in the electronic device 200 may be combined and inputted as a query into the user-customized information generator 210. The user-customized information generator 210 may readjust a parameter, a range, or a specific value contained in the initial device configuration information based on the personal information. For example, if the user-customized information generator 210 analyzes the patient personal information and discovers that the patient's current activity trend (e.g., an activity trend over recent several months) decreases in activity level, and a daily target step count designated by the primary device configuration information is about 5,000 steps, the user-customized information generator 210 may raise the target step count to about 6,000 steps to increase the patient's activity level. For example, if the personal information analysis results indicate that, although the wearable electronic device of the patient is a model with the body composition measurement function, the body composition measurement function has never been used, the user-customized information generator 210 may modify the body composition measurement cycle to once a week, and set a user classification flag indicating the patient is a first-time user for the body composition measurement function. For example, if the sleep start time designated by the initial device configuration information is 10 PM but the personal information analysis results indicate that an average sleep duration of the user is 7 hours and an average sleep start time is 11 PM, the user-customized information generator 210 may modify the sleep start time to approximately 11 PM or 10:30 PM.

According to an embodiment, the user-customized information generator 210 of the electronic device 200 may feed the self-readjusted device configuration information back to the digital prescription generator 310 of the external electronic device 300. The feedback device configuration information may be finally approved by the digital prescription generator 310 of the external electronic device 300 or by the doctor.

According to an embodiment, if there is no digital prescription issuance history for the patient (e.g., at the initial visit), the external electronic device 300 may issue a digital prescription by utilizing the personal information stored in the electronic device 200 of the patient, and then store the digital prescription issuance history in the local DB 320.

According to an embodiment, if there exists a digital prescription issuance history for the patient in the local DB 320 (e.g., at a next visit after the initial visit), the digital prescription generator 310 of the external electronic device 300 may, in conjunction with the user-customized information generator 210 of the electronic device 200, obtain various health activity result information such as participation and compliance levels, or goal achievement rate of the patient in relation to the previously issued digital prescription. Using the various health activity result information, the digital prescription generator 310 of the external electronic device 300 may provide the doctor with evolved user-customized advice, coaching, or guidance in issuing a subsequent digital prescription.

FIG. 5A is a flowchart illustrating an operation method of an electronic device 200 according to an embodiment.

The operation method of the electronic device 200 according to an embodiment may be to provide a user-customized healthcare function. For convenience of description, the method is assumed to be performed by the electronic device 200 of FIG. 2 and FIG. 3A. However, it is not limited thereto. For example, the method may be carried out by any one of the electronic device 101 of FIG. 1, the processor 120 of FIG. 1, the user-customized information generator 210 of FIG. 2, or the processor 270 of FIG. 3A.

Referring to FIG. 5A, the operation method of the electronic device 200 according to an embodiment may include operation 510, operation 520, operation 530, operation 540, operation 550 and operation 560.

In operation 510, the electronic device 200 (e.g., the smartphone 201) may receive an access request from the external electronic device 300 via the communication circuitry 250.

In operation 520, the electronic device 200 (e.g., the smartphone 201) may receive a user input for the access request from the external electronic device 300.

In operation 530, the electronic device 200 (e.g., the smartphone 201) may transmit the user's personal information stored in the electronic device 200 to the external electronic device 300 via the communication circuitry 250, based on the user input inputted in operation 520. In an embodiment, the personal information may include personal device information for at least one device of the user. In an embodiment, the personal information may further include personal health information (e.g., user profile information and/or health basis information) of the user.

For example, at least one device of the user may be registered in a healthcare application on the electronic device 200 (e.g., the smartphone 201). The electronic device 200 may store personal device information for the at least one device, and provide the personal device information to the external electronic device 300 during a medical consultation.

For example, the healthcare application on the electronic device 200 (e.g., the smartphone 201) may store user profile information and/or user health basis information collected from daily activities, and provide the user profile information and/or the health basis information to the external electronic device 300 at the medical treatment.

According to an embodiment, at least one device available for the user-customized healthcare function may include one or more of the smartphone 202, the wearable electronic device (e.g., the smartwatch 202), the IoT device 240, or the sensor 205.

According to an embodiment, at least one device available for the user-customized healthcare function may include at least one of the electronic device 200 (e.g., one of the smartphone 201 and the smartwatch 202) and one or more other electronic devices (e.g., the other of the smartphone 201 and the smartwatch 202, the sensor 205, or the IoT device 240).

According to an embodiment, at least one device available for the user-customized healthcare function may include a main device (e.g., the smartphone 201) and one or more peripheral devices (e.g., the wearable electronic device (e.g., the smartwatch 202), the IoT device 240, or the sensor 205).

In operation 540, the electronic device 200 (e.g., the smartphone 201) may receive device configuration information generated based on the personal information and digital prescription of the user from the external electronic device 300 via the communication circuitry 250.

In operation 550, the electronic device 200 (e.g., the smartphone 201) may generate healthcare guide information related to at least one device of the user based on the device configuration information received in operation 540.

In operation 560, the electronic device 200 (e.g., the smartphone 201) may display a user interface (e.g., a first screen 710 of FIG. 7) guiding the user's health activities on the display 260, based on the healthcare guide information generated in operation 550, and transmit a device control command for at least one device via the communication circuitry 250 to support the health activities.

According to an embodiment, the user interface may include information related to one or more of a measurement activity, an exercise activity, a sleep activity, scheduling, or guide content related to the user's health activities. The device control command may be to support the health activities by instructing a function to be executed on each of the at least one device.

For example, at least one device (e.g., a peripheral device, a wearable electronic device such as the smartwatch 202, the speaker 241, or the television 242) registered in the healthcare application on the electronic device 200 (e.g., the smartphone 201, the main device) each may, upon receiving the device control command from the electronic device 200 (e.g., the smartphone 201), interwork with the healthcare application of the electronic device 200 to output a user interface (e.g., at least one of an audio message 720, guide content 730, or a second screen 740 as shown in FIG. 7) to support the patient's health activities.

According to an embodiment, displaying the user interface and transmitting the device control command may be performed repeatedly over a designated period (e.g., for two weeks until the next visit, or at a designated time each day) based on scheduling information included in the healthcare guide information.

According to an embodiment, the electronic device 200 (e.g., the smartphone 201) may display a user interface guiding the user's initial health activities and collect actual health activity information of the user using at least one device (e.g., a wearable electronic device such as the smartwatch 202) of the user. The electronic device 200 may update the user interface based on the collected actual health activity information. The electronic device 200 may display the updated user interface, and transmit a device control command related to the updated user interface to the at least one device.

According to an embodiment, the electronic device 200 (e.g., the smartphone 201) may display on the display 260 a user interface inquiring about a privacy exposure level (e.g., whether to protect privacy, or a privacy protection level). For example, in response to the access request from the external electronic device 300, the screen of the electronic device 200 (e.g., the smartphone 201) may display a message such as "Would you like to apply privacy protection options if sharing your personal information with the hospital?". Based on a user input (e.g., a user input activating the privacy protection options) to the user interface, the electronic device 200 may transmit only some information filtered from the personal information of the user stored on the electronic device 200 according to the privacy exposure level, to the external electronic device 300. By means of this filtering, sharing of information with the high privacy level or sensitive level in the personal information may be restricted.

According to an embodiment, the electronic device 200 (e.g., the smartphone 201) may identify whether the electronic device 200 is located at a designated place (e.g., home). If located at the designated place, the electronic device 200 may display the user interface for healthcare guide information and transmit the device control command related to the healthcare guide information.

According to an embodiment, the electronic device 200 (e.g., the smartphone 201) may identify whether it is necessary to use an additional device not included in at least one device of the user based on the device configuration information received from the external electronic device 300. If it is necessary to use the additional device, the electronic device 200 may search for a place available with the additional device, and include and display an indicator (e.g., a map image showing a corresponding place) for guiding the user to the searched place within the user interface for the healthcare guide information. For example, if the user is a diabetic patient who must use a dedicated blood glucose meter for diabetes management but does not possess the dedicated blood glucose meter, the electronic device 200 may search for a place (e.g., a public health center or hospital) available with the dedicated blood glucose meter near the user's home, and provide the user with a user interface guiding to the searched place.

FIG. 5B is a flowchart illustrating an operation method of an external electronic device 300 according to an embodiment.

Referring to FIG. 5B, the operation method of the external electronic device 300 according to an embodiment may include operation 515, operation 525, operation 535, operation 545, and operation 555. For convenience of explanation, it is assumed that the method is performed by the external electronic device 300 of FIG. 2 and FIG. 3B. However, it is not limited thereto. For example, the method may be performed by any one of the server 108 of FIG. 1, the digital prescription generator 310 of FIG. 2, or the processor 370 of FIG. 3B.

In operation 515, the external electronic device 300 may generate a digital prescription (e.g., a primary digital prescription) based on medical information (e.g., medical examination, diagnostic results, treatment procedure details) inputted by the doctor.

In operation 525, the external electronic device 300 may transmit an access request to the electronic device 200 of the patient (or the user) via the communication circuitry 350.

In operation 535, the external electronic device 300 may receive personal information of the patient from the electronic device 200 via the communication circuitry 350. According to an embodiment, the personal information may include personal device information for at least one device. According to an embodiment, the personal information may further include personal health information (e.g., profile information and/or health basis information) of the patient.

In operation 545, the external electronic device 300 may generate device configuration information for a user-customized prescription based on the digital prescription generated in operation 515 and the personal information received in operation 535.

According to an embodiment, in operation 545, the external electronic device 300 may generate device configuration information for a user-customized prescription, based on the digital prescription (e.g., a primary digital prescription) and the patient's personal information (e.g., personal device information) received from the electronic device 200.

According to an embodiment, in operation 545, the external electronic device 300 may generate a digital prescription (e.g., a secondary digital prescription) modified from the initial digital prescription (e.g., a primary digital prescription), based on the patient's personal information (e.g., personal health information) received from the electronic device 200. Next, the external electronic device 300 may generate device configuration information for a user-customized prescription based on the generated digital prescription (e.g., the secondary digital prescription) and the personal information (e.g., personal device information).

In operation 555, the external electronic device 300 may transmit the device configuration information generated in operation 545 to the electronic device 200 of the patient via the communication circuitry 350.

According to an embodiment, the external electronic device 300 may determine whether any essential device required for the prescription (e.g., a dedicated blood glucose meter) is missing based on the digital prescription and the personal device information received from the electronic device 200 of the patient (or the user). If an essential device required for the prescription is missing, the external electronic device 300 may add information of the essential device to the device configuration information to be transmitted to the electronic device 200.

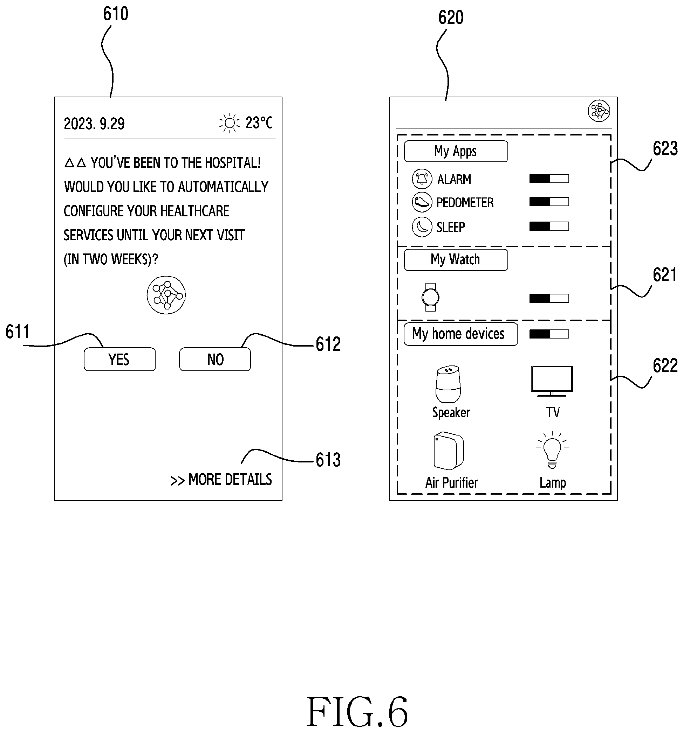

FIG. 6 is an example of a user interface for a user-customized healthcare function of an electronic device 200 according to an embodiment. The user interface in FIG. 6 may be to set a target for supporting the user-customized healthcare function. The electronic device 200 may have a healthcare application installed to provide the user-customized healthcare function. For example, the electronic device 200 may be the smartphone 201 of the user (or the patient).

According to an embodiment, if the user visits a hospital for treatment, the electronic device 200 of the user may provide personal device information of the user to the external electronic device 300.

Table 1 below shows the personal device information of the user. Referring to Table 1, the personal device information may include information related to at least some of a device ID, a device type, a model name, or whether to activate interworking with the healthcare application with respect to at least one device.

**[Table 1]**

| Device ID | Device Type | Model Name | Whether to enalbe interworking with Health App (ON/OFF) |
|---|---|---|---|
| 01 | smart phone | AA_1 | ON |
| 02 | smart watch | AA_2 | ON |
| 03 | smart band | AA_3 | OFF |
| 04 | smart phone_motion sensor | BB_1 | ON |
| 05 | smart watch_biometric sensor | BB_2 | ON |
| 06 | IoT device_home camera | CC_1 | OFF |
| 07 | IoT device_speaker | DD_1 | ON |
| 08 | IoT device_television | DD_2 | ON |
| 09 | IoT device_air purifier | DD_3 | OFF |

According to an embodiment, the external electronic device 300 may generate device configuration information for a user-customized prescription using the personal device information of the user as shown in Table 1, and then transmit the device configuration information to the electronic device 200. The electronic device 200 may store the device configuration information for the user-customized prescription, received from the external electronic device 300. The device configuration information may be information related to at least one device of the user. The at least one device may include at least one of various devices registered in the healthcare application of the electronic device 200, for example, the smartphone 201, the wearable electronic device (e.g., the smartwatch 202), the sensor 205, or the IoT device 240.

A first screen 610 and a second screen 620 shown in FIG. 6 may be user interfaces for setting a target device among at least one device of the user, for actually interworking with the healthcare application of the electronic device 200.

For example, after visiting a hospital and receiving treatment, the user may run the healthcare application which provides the user-customized healthcare function through the electronic device 200. As the healthcare application is executed, the electronic device 200 may display a user interface such as the first screen 610 shown in FIG. 6.

Referring to FIG. 6, the first screen 610 of the electronic device 200 may include menus 611 and 612 for inquiring about whether to activate automatic settings for at least one device of the user based on the device configuration information for the user-customized prescription received from the external electronic device 300. The user may approve the automatic settings by selecting the agree menu 611 on the first screen 610, or may reject the automatic settings by selecting the reject menu 612.

In an embodiment, if the user selects the agree menu 611, various devices (e.g., the smartwatch 202 or the IoT device 240) registered in the healthcare application may be automatically set as actual interworking targets of the healthcare application, for a designated period (e.g., from the present until the next scheduled medical appointment). In this case, the various devices may operate in conjunction with the healthcare application, to support the user-customized healthcare function.

In addition, if the user selects the agree menu 611, interworking between an application (e.g., an alarm application, a pedometer application, or a sleep care application) registered in the healthcare application and the healthcare application may be automatically set.

In an embodiment, if the user selects a more details menu 613 within the first screen 610, the first screen 610 may switch to the second screen 620.

Referring to FIG. 6, the second screen 620 of the electronic device 200 may include a first area 621 and a second area 622 showing a plurality of user devices registered in the healthcare application. The first area 621 is an area showing the registered wearable electronic device (e.g., the smartwatch 202), and may include an on/off menu for selecting whether to interoperate the wearable electronic device with the healthcare application. The second area 622 is an area showing the registered IoT device 240 (e.g., the speaker 241, the television 242, the air purifier 243, or the lamp 244), and may include an on/off menu for selecting whether to interoperate the IoT device 240 with the healthcare application.

In an embodiment, the second screen 620 of the electronic device 200 may further include a third area 623 showing applications (e.g., an alarm application, a pedometer application, or a sleep care application) for interworking with the healthcare application. The third area 623 may include an on/off menu for selecting whether to interworking each application with the healthcare application.

In an embodiment, the electronic device 200 may set the actual interworking target among the devices and/or the applications for interworking with the healthcare application based on a user input for the first screen 610 or the second screen 620.

For example, if the user selects the agree menu 611 on the first screen 610 to consent to the automatic settings for a plurality of devices and a plurality of applications registered in the healthcare service, the plurality of devices and the plurality of applications may be collectively set as the interworking targets for the healthcare application.

For example, if the user selects some of the plurality of devices and the plurality of applications on the second screen 620 using the on/off menu, only the selected ones may be set as targets for interworking with the healthcare application.

FIG. 7 is another example of a user interface for a user-customized healthcare function of an electronic device 200 according to an embodiment. FIG. 7 may illustrate displaying the user interface based on healthcare guide information and transmitting a device control command.

According to an embodiment, the electronic device 200 may be a smartphone 201 as shown in FIG. 7. A healthcare application for providing the user-customized healthcare function may be installed on the smartphone 201. However, the smartphone 201 and the user interface of the smartphone 201 in FIG. 7 are merely examples to help understanding, the electronic device 200 and the user interface outputted through the electronic device 200 are not limited thereto and may be applied, modified, and/or expanded in various manners.

Referring to FIG. 7, the smartphone 201 is a main device for providing the user-customized healthcare function, and at least one device (e.g., one or more of a smartwatch 202, a speaker 241, and a television 242) may be peripheral devices for supporting the user-customized healthcare function.

In an embodiment, the smartphone 201 may generate healthcare guide information related to at least one device of the user based on device configuration information received from an external electronic device 300. The smartphone 201 may display a user interface such as a first screen 710 of FIG. 7 to provide healthcare guide information to the user. For example, the healthcare guide information may include an indicator 711 showing an exercise goal for the day, an indicator 712 showing medication information, and an indicator 713 for guiding a necessary measurement activity.

In an embodiment, the smartphone 201 may set device control information for at least one device (e.g., one or more of the smartwatch 202, the speaker 241, and the television 242) to interwork with the healthcare application based on the device configuration information received from the external electronic device 300.

According to an embodiment, while the first screen 710 for the healthcare guide information is displayed through the smartphone 201, the smartphone 201 operating as the main device may transmit a device control command to at least one peripheral device (e.g., one or more of the smartwatch 202, the speaker 241, and the television 242) to support a user's health activity related to the healthcare guide information.

For example, if a blood pressure medication time (e.g., AM 9:00) included in the healthcare guide information arrives, the smartphone 201 may transmit a first device control command to the speaker 241. The first device control command may instruct the speaker 241 to output an audio message 720 notifying of the time to take blood pressure medication.

For example, if a blood pressure measurement time (e.g., 9:00 PM before sleeping) included in the healthcare guide information arrives, the smartphone 201 may transmit a second device control command to the television 242 and transmit a third device control command to the smartwatch 202. The second control command may instruct the television 242 to output guide content 730 for guiding a blood pressure measurement posture. The third control command may instruct the smartwatch 202 which is the wearable electronic device to display a second screen 740 for performing the blood pressure measurement, to activate a biosensor of the smartwatch 202 for a designated time, to thus wait for the blood pressure measurement of the user.

The user interfaces described in FIG. 6 and FIG. 7 are merely examples for explanation, and the scope of the embodiments of the disclosure is not limited thereto but may be applied, modified, and/or extended in various ways.

According to various embodiments, an electronic device 200 (e.g., the electronic device 200 of FIG. 2, FIG. 3A) may include communication circuitry (e.g., the communication circuitry 250 of FIG. 3A), memory (e.g., the memory 280 of FIG. 3A), a display (e.g., the display 260 of FIG. 3A), and at least one processor (e.g., the processor 270 of FIG. 3A). The memory may store instructions, when executed by the at least one processor, causing the electronic device to receive an access request from an external electronic device via the communication circuitry, receive a user input for the access request, transmit personal information to the external electronic device via the communication circuitry based on the user input, the personal information including personal device information for at least one device, receive device configuration information generated based on the personal information and a digital prescription, from the external electronic device via the communication circuitry, generate healthcare guide information related to the at least one device based on the device configuration information, and display a user interface for guiding a health activity on the display based on the healthcare guide information, and transmit a device control command to the at least one device via the communication circuitry 250 to support the health activity.

According to various embodiments, the at least one device may include at least one of the electronic device 200 and one or more other electronic devices.

According to various embodiments, the at least one device may include one or more of a smartphone, a wearable electronic device, an IoT device, or a sensor.

According to various embodiments, the user interface may include information of one or more of a measurement activity, an exercise activity, a sleep activity, scheduling, or guide content related to the health activity. The device control command may be to support the health activity by instructing a function to be executed on each of the at least one device.

According to various embodiments, displaying the user interface and transmitting the device control command may be repeatedly performed for a designated time based on scheduling information contained in the healthcare guide information.

According to various embodiments, the instructions may, when executed by the at least one processor, cause the electronic device to display a user interface for guiding an initial health activity of a user, collect actual health activity information of the user using the at least one device, and based on the collected actual health activity information, update the user interface, display the updated user interface, and transmit a device control command related to the updated user interface.

According to various embodiments, the instructions may, when executed by the at least one processor, cause the electronic device to display a user interface inquiring about a privacy exposure level on the display, and, based on a user input to the user interface, transmit only some information filtered based on the privacy exposure level from the personal information filtered, to the external electronic device.

According to various embodiments, the instructions may, when executed by the at least one processor, cause the electronic device to identify whether the electronic device is located at a designated place, and if the electronic device is located at the designated place, display the user interface and transmit the device control command.

According to various embodiments, the instructions may, when executed by the at least one processor, cause the electronic device to identify whether it is necessary to use an additional device not included in the at least one device, based on the device configuration information, if it is necessary to use the additional device, search for a place available with the additional device, and display an indicator guiding to the searched place within the user interface.

According to various embodiments, an external electronic device (e.g., the external electronic device 300 of FIG. 2, FIG. 3B) may include communication circuitry (e.g., the communication circuitry 350 of FIG. 3B), memory (e.g., the memory 380 of FIG. 3B) and at least one processor (e.g., the processor 370 of FIG. 3B). The memory may store instructions, when executed by the at least one processor, cause the external electronic device to generate a digital prescription based on inputted medical information, transmit an access request to the electronic device via the communication circuitry, receive personal information from the electronic device via the communication circuitry, the personal information including personal device information for at least one device, generate device configuration information for a user-customized prescription based on the personal information and the digital prescription, and transmit the device configuration information to the electronic device via the communication circuitry.

According to various embodiments, the instructions may, when executed by the at least one processor, cause the external electronic device to determine whether any essential device required for the prescription is missing based on the digital prescription and the personal device information, and, if the essential device is missing, add information of the essential device to the device configuration information.

An operation method of an electronic device (e.g., the electronic device 200 of FIG. 2, FIG. 3A) according to various embodiments may include receiving an access request from an external electronic device (e.g., the external electronic device 300 of FIG. 2, FIG. 3B), receiving a user input for the access request, transmitting personal information including personal device information for at least one device to the external electronic device based on the user input, receiving device configuration information generated based on the personal information and a digital prescription from the external electronic device, generating healthcare guide information related to the at least one device based on the device configuration information, and displaying a user interface for guiding a health activity based on the healthcare guide information, and transmitting a device control command to the at least one device to support the health activity.

According to various embodiments, the at least one device may include at least one of the electronic device and one or more other electronic devices.

According to various embodiments, the at least one device may include one or more of a smartphone, a wearable electronic device, an IoT device, or a sensor.

According to various embodiments, the user interface may include information of one or more of a measurement activity, an exercise activity, a sleep activity, scheduling, or guide content related to the health activity. The device control command may be to support the health activity by instructing a function to be executed on each of the at least one device.

According to various embodiments, displaying the user interface and transmitting the device control command may be repeatedly performed for a designated time based on scheduling information contained in the healthcare guide information.

According to various embodiments, displaying the user interface may include displaying a user interface for guiding an initial health activity of a user, collecting actual health activity information of the user using the at least one device, and, based on the collected actual health activity information, updating the user interface, displaying the updated user interface, and transmitting a device control command related to the updated user interface.

According to various embodiments, transmitting the personal information may include displaying a user interface inquiring about a privacy exposure level on the display, and, based on a user input to the user interface, transmitting only some information filtered based on the privacy exposure level from the personal information filtered, to the external electronic device.

According to various embodiments, the method may further include identifying whether the electronic device is located at a designated place. If the electronic device is located at the designated place, displaying the user interface and transmitting the device control command may be performed.

According to various embodiments, the method may further include identifying whether it is necessary to use an additional device not included in the at least one device, based on the device configuration information, if it is necessary to use the additional device, searching for a place available with the additional device, and displaying an indicator guiding to the searched place within the user interface.

An operation method of an external electronic device (e.g., the external electronic device 300 of FIG. 2, FIG. 3B) according to various embodiments may include generating a digital prescription based on inputted medical information, transmit an access request to an electronic device (e.g., the electronic device 200 of FIG. 2, FIG. 3A) via the communication circuitry, receiving personal information from the electronic device via the communication circuitry, the personal information including personal device information for at least one device, generating device configuration information for a user-customized prescription based on the personal information and the digital prescription, and transmitting the device configuration information to the electronic device via the communication circuitry.

According to various embodiments, the method may further include determining whether an essential device require for a prescription is missing based on the digital prescription and the personal device information, and, if the essential device is missing, adding information of the essential device to the device configuration information.

A computer-readable recording medium according to various embodiments may include may record a program for executing an operation method of an electronic device (e.g., the electronic device 200 of FIG. 2, FIG 3A), which includes receiving an access request from an external electronic device (e.g., the external electronic device 300 of FIG. 2, FIG. 3B), receiving a user input for the access request, transmitting personal information including personal device information for at least one device to the external electronic device based on the user input, receiving device configuration information generated based on the personal information and a digital prescription from the external electronic device, generating healthcare guide information related to the at least one device based on the device configuration information, and displaying a user interface for guiding a health activity based on the healthcare guide information, and transmitting a device control command to the at least one device to support the health activity.

A computer-readable recording medium according to various embodiments may include may record a program for executing an operation method of an external electronic device (e.g., the external electronic device 300 of FIG. 2, FIG. 3B), which includes generating a digital prescription based on inputted medical information, transmit an access request to an electronic device (e.g., the electronic device 200 of FIG. 2, FIG 3A) via the communication circuitry, receiving personal information from the electronic device via the communication circuitry, the personal information including personal device information for at least one device, generating device configuration information for a user-customized prescription based on the personal information and the digital prescription, and transmitting the device configuration information to the electronic device via the communication circuitry.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStoreTM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device 200 comprising:
communication circuitry 250;
memory 280;
a display 260; and
at least one processor 270,
wherein the memory 280 stores instructions, when executed by the at least one processor 270, causing the electronic device 200 to,
receive an access request from an external electronic device 300 via the communication circuitry 250,
receive a user input for the access request,
transmit personal information to the external electronic device 300 via the communication circuitry 250 based on the user input, the personal information comprising personal device information for at least one device,
receive device configuration information generated based on the personal information and a digital prescription, from the external electronic device 300 via the communication circuitry 250,
generate healthcare guide information related to the at least one device based on the device configuration information, and
display a user interface for guiding a health activity on the display 260 based on the healthcare guide information, and transmit a device control command to the at least one device via the communication circuitry 250 to support the health activity.

2. The electronic device of claim 1, wherein the at least one device comprises at least one of the electronic device 200 and one or more other electronic devices.

3. The electronic device of claim 1, wherein the at least one device comprises one or more of a smartphone, a wearable electronic device, an internet of things (IoT) device, or a sensor.

4. The electronic device of claim 1, wherein the user interface comprises information of one or more of a measurement activity, an exercise activity, a sleep activity, scheduling, or guide content related to the health activity, and
the device control command is to support the health activity by instructing a function to be executed on each of the at least one device.

5. The electronic device of claim 1, wherein displaying the user interface and transmitting the device control command are repeatedly performed for a designated time based on scheduling information contained in the healthcare guide information.

6. The electronic device of claim 1, wherein the instructions, when executed by the at least one processor 270, cause the electronic device 200 to,
display a user interface for guiding an initial health activity of a user,
collect actual health activity information of the user using the at least one device, and
based on the collected actual health activity information, update the user interface, display the updated user interface, and transmit a device control command related to the updated user interface.

7. The electronic device of claim 1, wherein the instructions, when executed by the at least one processor 270, cause the electronic device 200 to,
display a user interface inquiring about a privacy exposure level on the display, and
based on a user input to the user interface, transmit only some information filtered based on the privacy exposure level from the personal information filtered, to the external electronic device.

8. The electronic device of claim 1, wherein the instructions, when executed by the at least one processor 270, cause the electronic device 200 to,
identify whether the electronic device is located at a designated place, and
if the electronic device is located at the designated place, display the user interface and transmit the device control command.

9. The electronic device of claim 1, wherein the instructions, when executed by the at least one processor 270, cause the electronic device 200 to,
identify whether it is necessary to use an additional device not included in the at least one device, based on the device configuration information,
if it is necessary to use the additional device, search for a place available with the additional device, and
display an indicator guiding to the searched place within the user interface.

10. An external electronic device 300 comprising:
communication circuitry 350;
memory 380; and
at least one processor 370,
wherein the memory 380 stores instructions, when executed by the at least one processor 370, cause the external electronic device 300 to,
generate a digital prescription based on inputted medical information,
transmit an access request to an electronic device 200 via the communication circuitry 350,
receive personal information from the electronic device 200 via the communication circuitry 350, the personal information comprising personal device information for at least one device,
generate device configuration information for a user-customized prescription based on the personal information and the digital prescription, and
transmit the device configuration information to the electronic device 200 via the communication circuitry 350.

11. The external electronic device of claim 10, wherein the instructions, when executed by the at least one processor 370, cause the external electronic device 300 to,
determine whether any essential device required for the prescription is missing based on the digital prescription and the personal device information, and
if the essential device is missing, add information of the essential device to the device configuration information.

12. An operation method of an electronic device, comprising:
receiving an access request from an external electronic device;
receiving a user input for the access request;
transmitting personal information comprising personal device information for at least one device to the external electronic device based on the user input;
receiving device configuration information generated based on the personal information and a digital prescription from the external electronic device;
generating healthcare guide information related to the at least one device based on the device configuration information; and
displaying a user interface for guiding a health activity based on the healthcare guide information, and transmitting a device control command to the at least one device to support the health activity.

13. The method of claim 12, wherein the at least one device comprises at least one of the electronic device and one or more other electronic devices.

14. The method of claim 12, wherein the at least one device comprises one or more of a smartphone, a wearable electronic device, an internet of things (IoT) device, or a sensor.

15. The method of claim 12, wherein the user interface comprises information of one or more of a measurement activity, an exercise activity, a sleep activity, scheduling, or guide content related to the health activity, and
the device control command is to support the health activity by instructing a function to be executed on each of the at least one device.
